# EUROPÄISCHE PATENTSCHRIFT

(11) **EP 1 763 379 B1**
(45) Veröffentlichungstag und Bekanntmachung des Hinweises auf die Patenterteilung: **02.09.2009**
(21) Anmeldenummer: 05769369.9
(22) Anmeldetag: 30.06.2005
(51) Int. Cl.: A61N 5/06, A61H 39/00

(54) **LASERNADEL ZUR DURCHFÜHRUNG EINER KOMBINIERTEN LASERNADEL-ELEKTROAKUPUNKTUR**
LASER NEEDLE FOR PERFORMING A COMBINED LASER NEEDLE/ELECTRIC ACUPUNCTURE
AIGUILLE LASER PERMETTANT DE PRATIQUER UNE ACUPONCTURE COMBINEE AIGUILLE LASER / ELECTRIQUE

(30) Priorität: 03.07.2004 DE 102004032394
(43) Veröffentlichungstag der Anmeldung: 21.03.2007
(73) Patentinhaber: Weber, Michael, 37697 Lauenförde (DE)
(72) Erfinder: Weber, Michael, 37697 Lauenförde (DE)
(74) Vertreter: Seewald, Jürgen
(86) Internationale Anmeldenummer: PCT/DE2005/001157
(87) Internationale Veröffentlichungsnummer: WO 2006/005298

(56) Entgegenhaltungen:
- EP-A- 1 337 298
- DE-U1- 20 309 976
- DE-U1- 29 519 433
- US-A- 5 304 207

## Beschreibung

Die vorliegende Erfindung betrifft eine Lasernadel gemäß dem Oberbegriff des Anspruchs 1.

Eine gattungsgemäße Lasernadel ist aus DE 203 09 976 U1 bekannt. Dieses Gebrauchsmuster beschreibt eine neue Vorrichtung zur Akupunktur mittels Laserstrahlung. Bei dieser wie auch bei allen anderen bekannten Vorrichtungen zur Laserakupunktur müssen die Endstücke, so genannte Lasernadeln, in einen unmittelbaren Kontakt mit der Haut eines Patienten gebracht werden, um eine optimale Einkopplung der Laserstrahlung in das Gewebe zu ermöglichen. Dadurch wird eine hohe Energiedichte am Akupunkturpunkt sichergestellt.

In dem oben genannten Gebrauchsmuster ist auch eine Lasernadel zur kombinierten Lasernadel-Elektroakupunktur beschrieben. Diese Lasernadel weist eine Lichtleitfaser, z.B. eine Glasfaser oder eine glasfaseranaloge Kunststofffaser, auf, die von einer stromführenden Hülse umgeben ist. Über diese Hülse wird zusätzlich zu dem über die Lichtleitfaser in das Gewebe eingebrachten Laserstrahl Reizstrom in die Haut bzw. das Gewebe eines Patienten übertragen. Diese Lasernadel ist in der Fachwelt unter der Bezeichnung "Weberneedle" bekannt geworden.

In EP 1 337 298 A1 ist eine weitere Vorrichtung zur Akupunktur mittels Laserstrahlung beschrieben. Die im Zusammenhang mit dieser Vorrichtung verwendete Lasernadel ist für eine Elektroakupunktur nicht geeignet. Zur Befestigung der Lasernadel am Körper eines Patienten ist ein auf dem Gehäuse der Lasernadel verschiebbares Befestigungselement vorgesehen. Dieses besteht in einer offenbarten Ausführungsform aus einem Schaft, der auf das Gehäuse der Lasernadel aufschiebbar ist und an seinem unteren Ende in einen Teller übergeht. Das Befestigungselement besteht aus Gummi und wird durch Hafttreibung zwischen dem Schaft und dem Gehäuse an der Lasernadel festgehalten. In einer anderen Ausführungsform erfolgt die Befestigung des Befestigungselementes an der Lasernadel über eine Klammer. An der Unterseite des Tellers ist ein doppelt wirkender Klebering angeordnet, der der Anbringungen des Befestigungselementes und damit der Lasernadel am Körper eines Patienten dient.

Es ist wissenschaftlich bewiesen, dass sowohl die Lasernadelakupunktur als auch die Elektroakupunktur an spezifischen Punkten des menschlichen Körpers nachweisbare Veränderungen am Nervensystem erzeugen (Verbesserung der Sauerstoffversorgung, des Blutflusses und der Stoffwechselaktivität). Es kann somit davon ausgegangen werden, dass ein Applikation von Laserlicht kombiniert mit Reizstrom an multiplen Punkten simultan einen erheblich gesteigerten Effekt der Behandlung bewirkt und somit die Behandlungseffektivität des angewendeten Systems erheblich verbessert wird. Eine kombinierte Lasernadel-Elektroakupunktur ist daher von erheblicher medizinischer und gesundheitspolitischer Relevanz.

Vor diesem Hintergrund ist es Aufgabe der vorliegenden Erfindung, die bekannte Lasernadel zur kombinierten Lasernadel-Elektroakupunktur in ihrer Effizienz noch zu verbessern.

Diese Aufgabe wird erfindungsgemäß mit einer Lasernadel gelöst, die die Merkmale des Anspruchs 1 aufweist.

Dabei kann der Teller in vorteilhafter Ausgestaltung der Erfindung entweder einstückig mit der Hülse ausgebildet sein, oder aber auch mit einem Schaft versehen sein, der über die Hülse schiebbar und kraft- oder formschlüssig und stromleitend an dieser festlegbar ist.

Die breitere Auflagefläche des Tellers hat gegenüber der bekannten Laser-Elektroakupunktur-Nadel den Vorteil, dass der Reizstrom breitflächig auf dem Gewebe verteilt wird. Über die große Auflagefläche können höhere Stromdosen zugeführt werden, ohne dass es zu einer Überhitzungen des Gewebes kommt. Weiterhin ist die Effektivität des Reizstromflusses auf der Haut und letztlich die Akupunkturwirkung deutlich erhöht, wenn eine etwas breitflächigere Auflage besteht. Dabei kann die Effektivität des Stromeindringens in die Haut noch dadurch erhöht werden, dass eine leitende Flüssigkeit oder ein leitendes Gel zwischen die Unterseite des Tellers und die Haut gebracht wird.

Gleichzeitig dient der Teller der Befestigung der Lasernadel am Körper eines Patienten. Dazu bieten sich mehrere Möglichkeiten an.

In einer Ausführungsform der Erfindung erfolgt die Befestigung der Lasernadel mittels eines Lochpflasters, das mit seinem Loch über die Hülse bzw. den Schaft des Tellers schiebbar ist, so dass es von oben auf dem Teller aufliegt und seine Klebeschicht den Teller seitlich überragt. Die dadurch von der Längsachse der Lasernadel relativ weit entfernten Klebflächen sowie die relativ große Auflagefläche des Tellers sorgen für einen sicheren Halt der Lasernadel auf der Haut eines Patienten.

In einer anderen Ausführungsform der Erfindung erfolgt die Befestigung der Lasernadel am Körper eines Patienten durch Unterdruck. Dazu ist der Teller perforiert und mit einem aufgesetzten Saughütchen ausgestattet, welches über eine Saugleitung zur Erzeugung des Unterdrucks evakuierbar ist.

Es versteht sich, dass diese Befestigungsmöglichkeiten mit einem Lochpflaster und einem Saughütchen auch auf einfache Lasernadeln, d.h. Lasernadeln, mit denen keine Elektroakupunktur durchgeführt werden kann, übertragbar sind.

Es ist in Ausgestaltung der Erfindung weiterhin von Vorteil, wenn der Teller und sein Schaft aus Edelstahl bestehen. Dieses Material garantiert nicht nur eine gute Stromleitung, sondern nach der Behandlung kann es in einem Dampf- oder Heißluftsterilisator keimfrei gemacht und wieder verwendet werden. Somit ist für die Akupunkturbehandlung eine optimale Hygiene gewährleistet.

Weitere Ausgestaltungen der Erfindung ergeben sich aus den übrigen Unteransprüchen. Die Erfindung wird nachstehend anhand von Ausführungsbeispielen näher erläutert. In der dazugehörigen Zeichnung zeigen schematisch:
- Fig. 1-6: verschiedene Ausführungsformen, bei denen der Teller über seinen Schaft und Federmittel an der Lasernadel festge- klemmt ist,
- Fig. 7: eine Befestigung des Tellers an der Lasernadel durch Ver- schraubung,
- Fig. 8: einen einstückig mit der stomführenden Hülse der Laserna- del ausgeführten Teller,
- Fig. 9: eine Ausführung mit einem auf den Teller aufgesetzten Saughütchen,
- Fig. 10: eine Darstellung gemäß Fig. 2 mit verkleinerter Stromappli- kationsfläche,
- Fig. 11: eine Darstellung gemäß Fig. 9 mit verkleinerter Stromappli- kationsfläche, und
- Fig. 12: verschiedene Tellerformen.

Vorrichtungen zur Durchführung einer kombinierten Lasernadel-Elektroakupunktur weisen Endstücke, so genannten Lasernadeln 1 auf, die neben einer Lichtleitfaser 2 noch eine diese umgebende, stromleitende Metallhülse 3 besitzen. In die Lichtleitfaser 2 wird über eine nicht dargestellte Laserdiode ein Laserstrahl eingekoppelt, der über die Lichtleitfaser 2 direkt über deren Ausgangsbereich 4, der sich in einem unmittelbaren Kontakt mit der nicht dargestellten Haut eines Patienten befindet optimal in das Gewebe eingetragen wird. Die Lichtleitfaser 2 befindet sich in einer schützenden Kunststoffhülle, in der gleichzeitig eine Stromzuführung zur Stromversorgung der Hülse 3 mitgeführt ist. Insoweit entspricht die bisher beschriebene Lasernadel 1 der aus dem Stand der Technik bekannten Laser-Elektroakupunktur-Nadel.

Zur Erhöhung der Effizienz der Laser-Elektroakupunktur-Nadeln und zur Verbesserung der Befestigung derartiger Nadeln auf dem Körper eines Patienten sind Teller 5 vorgesehen, durch die der Reizstrom für die Elektroakupunktur auf eine größere Fläche verteilt wird.

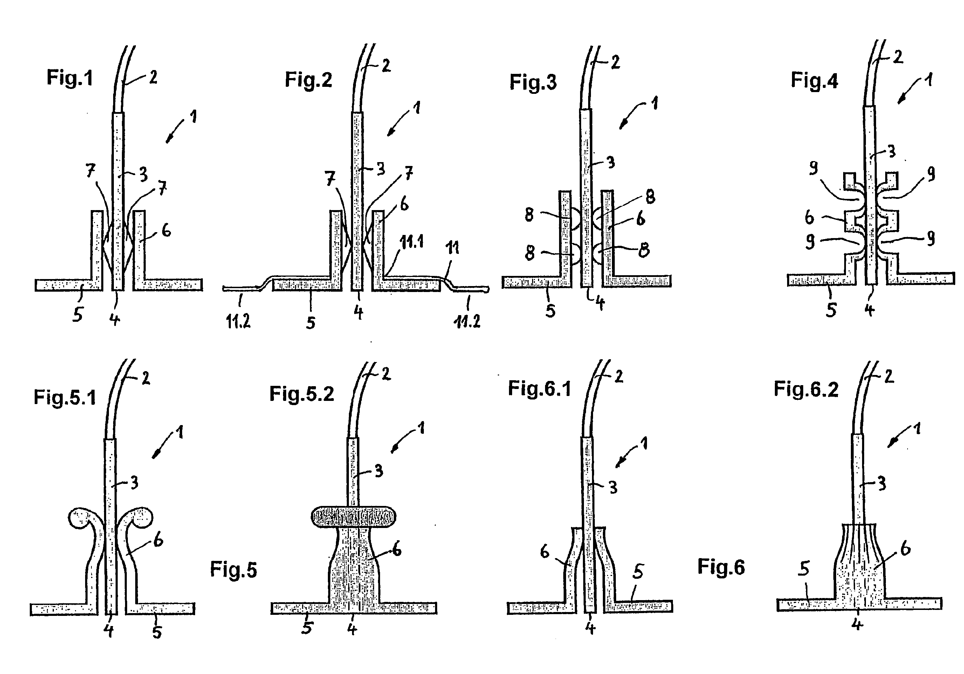

In den Ausführungsformen gemäß den Figuren 1 bis 7, 10 und 12 ist einstückig mit dem Teller 5 ein Schaft 6 ausgebildet, der der Befestigung des Tellers 5 an der Hülse 3 der Lasernadel 1 dient. In den Ausführungsbeispielen gemäß den Fig. 1 bis 6 erfolgt die Befestigung über Federmittel, die gleichzeitig eine stromleitenden Verbindung zwischen der Hülse 3 und dem Teller 5 herstellen. Im Falle des Ausführungsbeispieles gemäß Fig. 1 sind Federzungen 7 einstückig mit der Hülse 3 ausgebildet. Beim Aufschieben des Schaftes 6 auf die Hülse 3 federn diese Federzungen 7 etwas ein und klemmen dann aufgrund des Federdrucks den Schaft 6 und damit auch den Teller 5 an der Hülse 3 fest. Bei dem Ausführungsbeispiel gemäß Fig. 2 sind die Federzungen 7 in Umkehrung des vorhergehenden Beispiels an den Schaft 6 ausgebildet.

Die Figuren 3 bis 6 zeigen weitere Varianten, bei denen der Schaft 6 durch Federdruck an der Hülse 3 festgelegt ist. Im Falle des Ausführungsbeispieles gemäß Fig.3 sorgen in dem Schaft 6 federnd gelagerte Kugeln 8 für den notwendigen Anpressdruck. In dem Bespiel gemäß Fig. 4 ist der Schaft 6 mit Eindrückungen 9 versehen, über die der notwendige Federdruck zur Verfügung gestellt wird.

In den Ausführungsbeispielen gemäß den Figuren 5 und 6, in denen die Figuren 5.1 bzw. 6.1 Schnittdarstellungen und die Figuren 5.2 und 6.2 Volldarstellungen zeigen, sind die Schäfte 6 an ihren oberen Enden nach innen eingezogen, so dass sich auch hier eine federnde Klemmwirkung ergibt.

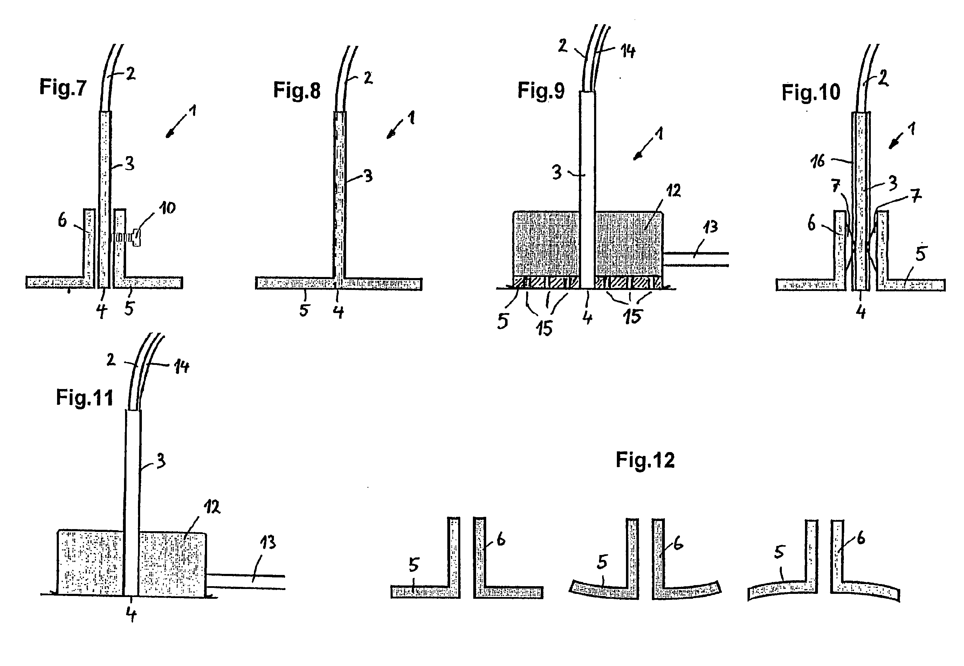

In dem in Fig. 7 gezeigten Ausführungsbeispiel ist der Schaft 6 mittels einer Madenschraube 10 an der Hülse 3 befestigt. Auch hier ist über die Madenschraube 10 eine elektrisch leitende Verbindung zur Hülse 3 hergestellt.

Fig. 8 zeigt eine Ausführung, bei der die Hülse 3 und der Teller 5 einstückig ausgebildet sind.

Bei den oben beschriebenen Ausführungsformen der Erfindung erfolgt die Befestigung der Lasernadel 1 am Körper eines Patienten mittels eines Lochpflasters 11, welches nur in Fig. 2 exemplarisch eingezeichnet ist. Das Lochpflaster 11 wird mit seinem mittigen Loch 11.1 über den Schaft 6 gezogen und liegt dann von oben auf dem Teller 5 auf, die den Teller 5 überragenden Ränder 11.2 des Lochpflasters 11 dienen dem Ankleben auf der Haut eines Patienten. Aufgrund der breiten Basis des Tellers 5 und der seitlich relativ weit von der zentralen Achse der Lasernadel 1 entfernten Klebestellen 11.2 ist eine sichere Befestigung der Lasernadel 1 am menschlichen Körper gewährleistet.

In dem Ausführungsbeispiel gemäß Fig. 9 ist eine weitere Befestigungsmöglichkeit für eine Lasernadel 1 dargestellt. Hier ist bei der Ausführungsform gemäß Fig. 8 auf die Oberseite des Tellers 5 ein Saughütchen 12 aufgesetzt. Dieses Saughütchen 12 ist über Saugleitungen 13, 14 evakuierbar, wobei die Sauleitung 14 mit der Lichtleitfaser 2 von oben in das Saughütchen 12 eingebracht wird, während die Saugleitung 13 separat in das Saughütchen 12 eingeführt wird. Im Ausführungsbeispiel gemäß Fig. 9 sind beide Saugleitungen 13, 14 gleichzeitig eingezeichnet. Es versteht sich, dass in der Praxis jeweils nur eine dieser Saugleitungen 13, 14 zum Einsatz kommt. Der Teller 5 besitzt Perforationen in Form von Durchgangbohrungen 15, über die der im Saughütchen erzeugte Unterdruck auf die Haut eines Patienten wirkt und somit für ein sicheres Ansaugen des Tellers 5 an die Haut saugen.

In Fig. 11 ist eine Variante der Ausführungsform gemäß Fig. 9 gezeigt. Bei dieser Variante, welche keinen Teil der Erfindung bildet, erfolgt die Reizstromzuführung nur über die kleine Stirnfläche der Hülse 3. Diese Reizstromapplikation ist sinnvoll, wenn in bestimmten Anwendungen ein hoher Stromfluss auf kleiner Fläche erzeugt werden muss. Auch bei diesem Beispiel sind die beiden Saugleitungen 13, 14 exemplarisch gleichzeitig eingezeichnet.

Die Ausführung gemäß Fig. 10 ist eine Variation des in Fig. 2 gezeigten Ausführungsbeispiels. Hier ist über die Hülse 3 eine Kunststoffhülse 16 gezogen, wodurch der Schaft 6 und damit der Teller 5 gegenüber der stromführenden Hülse 3 elektrisch isoliert sind. Diese Variante entspricht damit in der Reizstromapplikation dem Ausführungsbeispiel gemäß Fig. 11.

In Fig. 12 sind verschiedene Formen des Tellers 5 dargestellt, um eine optimale Anpassung an Körperformen zu gewährleisten. So kann der Teller 5 gerade, konvex oder auch konkav geformt sein. Hier kann es sinnvoll sein, einem behandelnden Arzt ein Sortiment derartiger Teller an die Hand zu geben, damit dieser je nach Akupunkturpunkt einen Teller mit passender Form auswählen kann.

## Patentansprüche

1. Lasernadel zur Durchführung einer kombinierten Lasernadel-Elektroakupunktur mit einer Lichtleitfaser (2) und einer diese umgebenden, stromführenden Hülse (3), über die zusätzlich zu einem über die Lichtleitfaser (2) applizierten Laserstrahl Reizstrom ist das Gewebe eines Patienten applizierbar ist, **dadurch gekennzeichnet, dass** die Hülse (3) an ihrem unteren Ende zur Verteilung des Reizstroms auf eine größere Fläche in einen Teller (5) übergeht, der gleichzeitig der Befestigung der Lasernadel am Körper eines Patienten dient.

2. Lasernadel nach Anspruch 1, **dadurch gekennzeichnet, dass** der Teller (5) einstückig mit der Hülse (3) ausgebildet ist.

3. Lasernadel nah Anspruch 1, **dadurch gekennzeichnet, dass** der Teller (5) mit einem auf die Hülse (3) aufschiebbaren Schaft (6) versehen ist, der kraft- oder formschlüssig und stromleitend an der Hülse (3) festlegbar ist.

4. Lasernadel nach Anspruch 3, **dadurch gekennzeichnet, dass** Schaft (6) und Teller (5) aus Edelstahl bestehen.

5. Lasernadel nach einem der vorstehenden Ansprüche, **dadurch gekennzeichnet, dass** zu ihrer Befestigung ein mit einem Loch (11.1) versehenes Lochpflaster (11) über die Hülse (3) bzw. den Schaft (6) schiebbar ist, dessen Klebeschicht (11.2) den Teller (5) seitlich überragt.

6. Lasernadel nach einem der Ansprüche 1 bis 4, **dadurch gekennzeichnet, dass** der Teller (5) perforiert und mit einem aufgesetztem Saughütchen (12) ausgestattet ist, welches über eine Saugleitung (13, 14) zur Erzeugung eines Unterdrucks evakuierbar ist.

7. Lasernadel nach einem der vorstehenden Ansprüche, **dadurch gekennzeichnet, dass** der Teller (5) gerade, konvex oder konkav geformt ist.

## Claims

1. Laser needle for,performing a combined laser needle/electric acupuncture with an optical fibre (2) and a current-carrying sheath (3) surrounding this, via which sheath, in addition to a laser beam applied via the optical fibre (2), stimulation current can be applied to the tissue of a patient, **characterized in that** the sheath (3), to distribute the stimulation current to a larger area, passes at its lower end into a plate (5), which serves at the same time to attach the laser needle to a patient's body.

2. Laser needle according to claim 1, **characterized in that** the plate (5) is formed in one piece with the sheath (3).

3. Laser needle according to claim 1, **characterized in that** the plate (5) is provided with a shaft (6), which can be pushed onto the sheath (3) and can be fixed on the sheath (3) in a force-fitting or form-fitting and current-carrying manner.

4. Laser needle according to claim 3, **characterized in that** shaft (6) and plate (5) consist of stainless steel.

5. Laser needle according to one of the preceding claims, **characterized in that** to attach it a perforated plaster (11) provided with a hole (11.1) can be pushed over the sheath (3) and the shaft (6), the adhesive layer (11.2) of the plaster protruding laterally beyond the plate (5).

6. Laser needle according to one of claims 1 to 4, **characterized in that** the plate (5) is perforated and fitted with a suction cap (12) placed thereon, which can be evacuated via a suction line (13, 14) to create a vacuum.

7. Laser needle according to one of the preceding claims, **characterized in that** the plate (5) is shaped to be straight, convex or concave.

## Revendications

1. Aiguille laser destinée à l'exécution d'une électro-acupuncture combinée à une acupuncture par aiguille laser, comprenant une fibre optique (2) entourée d'une gaine (3) parcourue par le courant, fibre optique (2), par l'intermédiaire de laquelle il est possible d'appliquer un courant de stimulation dans les tissus d'un patient en plus d'un rayon laser appliqué par l'intermédiaire de la fibre optique (2) **caractérisée en ce que** pour la répartition du courant de stimulation sur une surface plus importante, la gaine (3) se transforme, au niveau de son extrémité inférieure, en un disque (5), qui sert en même temps à la fixation de l'aiguille laser sur le corps d'un patient.

2. Aiguille laser suivant la revendication 1, **caractérisée en ce que** le disque (5) est conçu de manière à ne former qu'une seule pièce avec la gaine (3).

3. Aiguille laser suivant la revendication 1, **caractérisée en ce que** le disque (5) est pourvu d'une queue (6), que l'on peut faire glisser sur la gaine (3) et fixer sur celle-ci par adhérence ou liaison mécanique et de manière à conduire le courant.

4. Aiguille laser suivant la revendication 3, **caractérisée en ce que** la queue (6) et le disque (5) sont en acier inoxydable.

5. Aiguille laser suivant une des revendications précédentes, **caractérisée en ce que** pour la fixation de celle-ci, un pansement perforé (11) présentant un trou (11.1) peut être passé sur la gaine (3) ou la queue (6), pansement perforé (11), dont la couche adhésive (11.2) fait saillie latéralement du disque (5).

6. Aiguille laser suivant une des revendications 1 à 4, **caractérisée en ce que** le disque (5) est perforé et équipé d'une petite calotte aspirante (12) posée permettant d'évacuer l'air via une conduite aspirante (13, 14) servant à produire un vide.

7. Aiguille laser suivant une des revendications précédentes, **caractérisée en ce que** le disque (5) présente une forme plate, convexe ou concave.
